# EUROPEAN PATENT APPLICATION

(11) **EP 4 702 922 A1**
(43) Date of publication of application: **04.03.2026**
(21) Application number: 24197899.8
(22) Date of filing: 02.09.2024
(51) Int. Cl.: A61B 5/16, A61B 5/246, A61B 5/374, A61B 5/377, A61B 5/38, A61B 5/00

(54) **A BRAIN SCANNING SYSTEM FOR NON-INVASIVE DETECTION OF ELECTROMAGNETIC RESPONSES**

(71) Applicant: Psytech B.V., 2312 NR Leiden (NL)
(72) Inventor: RUDMAN, Mykhailo, 2312 NR Leiden (NL); RUDMAN, Dmytro, 2312 NR Leiden (NL)
(74) Representative: DeltaPatents B.V.

(57) **Abstract**

Some embodiments are directed to a brain scanning system including one or more noise generators configured to generate an electromagnetic noise field, the noise generator being arranged for positioning in proximity to the subject's head, wherein during operation of the brain scanning system the generated electromagnetic noise field interacts with the brain's electromagnetic field, resulting in an interfered noise signal.

## Description

### TECHNICAL FIELD

The presently disclosed subject matter relates to a brain scanning system, a noise generator, a brain scanning method, and a computer storage medium.

### BACKGROUND

The paper "Personalized brain circuit scores identify clinically distinct biotypes in depression and anxiety", by Leonardo Tozzi et al. describes a system using a magnetic resonance imaging protocol to derive quantitative measures for neurobiological dysfunctions or 'biotypes' to enable stratification of patients.

Participants underwent the Stanford Et Cere Image Processing System protocol, which probes several brain circuits. The biotypes were identified by a hierarchical clustering of regional circuit scores. Unfortunately, magnetic resonance imaging is both resource intensive, less accessible, and lacks a high temporal resolution.

An alternative approach to obtaining information on the active brain is the electroencephalogram (EEG). The EEG is a non-invasive method used to record electrical activity of the brain. This technique involves the placement of multiple electrodes on the scalp, which detect and measure the voltage fluctuations resulting from ionic current flows within the neurons of the brain. These fluctuations are indicative of neural activity and are recorded as waveforms, which are then analyzed to identify various brain states such as wakefulness, sleep stages, and abnormalities like epileptic seizures. EEG provides high temporal resolution, making it ideal for capturing rapid changes in brain activity. EEG electrodes are commonly made from conductive materials such as silver/silver chloride and are attached to the scalp using conductive gel or saline solutions to ensure proper contact.

A known EEG system is described in US patent application US 2023/0225659. The known system shows an earbud including a circuit, one or more speakers, one or more microphones, and a battery. The earbud measures an electroencephalogram (EEG), for measuring brain activity. A set of electrodes are disposed on a tip of the earbud, including active electrodes and reference electrodes. For measuring the EEG, an impedance between an active electrode and a reference electrode is measured.

Known systems for measuring an EEG, including the known system rely on a conductive EEG sensor which needs to make direct contact with the scalp. There is a need for alternative sensing principles.

### SUMMARY

A brain scanning system is presented which is configured for non-invasive detection of electromagnetic responses generated by a human brain of a subject. The brain scanning system may comprise stimulus generator and one or more noise generators. The stimulus generator is configured to present an auditory stimulus to the subject during or before scanning of the subject. The one or more noise generators are configured to generate an electromagnetic noise field. The noise generator is arranged for positioning in proximity to the subject's head, wherein during operation of the brain scanning system the generated electromagnetic noise field interacts with the brain's electromagnetic field, resulting in an interfered noise signal.

A further aspect is a brain scanning method. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, electronic storage, flash and disk storage, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

### BRIEF DESCRIPTION OF DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Figure 1 schematically shows an example of an embodiment of a brain scanning system,
Figure 2a schematically shows an example of an embodiment of a brain scanning system,
Figure 2b schematically shows an example of an embodiment of a brain scanning system,
Figure 2c schematically shows an example of an embodiment of a brain signal processing system,
Figure 2d schematically shows an example of an embodiment of a brain signal processing system,
Figure 3 schematically shows an example of an embodiment of a noise generator,
Figure 4 schematically shows an example of an embodiment of a brain scanning method,
Figure 5a schematically shows a computer readable medium having a writable part comprising a computer program according to an embodiment,
Figure 5b schematically shows a representation of a processor system according to an embodiment.

### Reference signs list

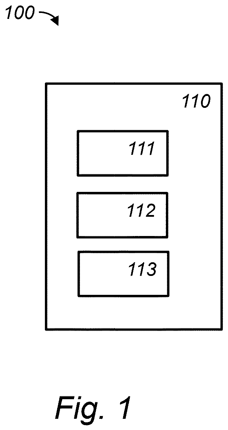

The following list of references and abbreviations corresponds to figures 1-3, and 5a-5b, and is provided for facilitating the interpretation of the drawings and shall not be construed as limiting the claims.
- 110: a brain scanning system
- 111: a processor system
- 112: a storage
- 113: a communication interface
- 200-201: a brain scanning system
- 202-203: a processing system
- 210: a stimulus generator
- 221, 222: a noise generator
- 223: a reference noise generator
- 224: a noise generator electromagnetic noise field
- 260: a subject
- 261: a subject electromagnetic field
- 225: an interfered electromagnetic noise field
- 231-233: an analog-to-digital converter
- 235, 236: a digital sequence
- 237: a reference digital sequence
- 240: a frequency processing unit
- 241-243: a series of bands
- 250: a correlation unit
- 251: a reference database
- 252: a correlation result
- 300: a noise generator
- 310: an interference injection point
- 311, 312: a transistor
- 320: an amplification connection
- 330: a power connection

- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

**Figure 1** schematically shows an example of an embodiment of a brain scanning device 110. Brain scanning device 110 is configured for non-invasive detection of electromagnetic responses generated by a human brain of a subject. The device can non-invasively detect electromagnetic responses generated by the human brain in response to auditory stimuli. Typically, the subject is a human subject. The subject is alive and the subject's brain is responsive to auditory stimuli and capable of generating detectable electromagnetic responses when exposed to such stimuli.

The system includes a stimulus generator that presents an auditory stimulus to the subject during or before scanning, and one or more noise generators that produce an electromagnetic noise field positioned near the subject's head. This noise field interacts with the brain's electromagnetic field, creating an interfered noise signal. The processed interfered noise signal is correlated with a database of reference samples. Interestingly, by generating different stimuli, the system can obtain a great variety of data. Furthermore, embodiments of the noise generator can be created at low costs.

Brain scanning device 110 may comprise a processor system 111, a storage 112, and a communication interface 113.

Brain scanning devices 110 is represented here as a single device, though it could just as well be implemented as systems, e.g., a geographically distributed system, e.g., a cloud computing system, e.g., a system comprising multiple computers.

Storage 112 may be, e.g., electronic storage, magnetic storage, etc. The storage may comprise local storage, e.g., a local hard drive or electronic memory. Storage 112 may comprise non-local storage, e.g., cloud storage. In the latter case, storage 112 may comprise a storage interface to the non-local storage. Storage may comprise multiple discrete sub-storages together making up storage 112

Storage 112 may be non-transitory storage. For example, storage 112 may store data in the presence of power such as a volatile memory device, e.g., a Random Access Memory (RAM). For example, storage 112 may store data in the presence of power as well as outside the presence of power such as a non-volatile memory device, e.g., Flash memory. Storage 112 may comprise a volatile writable part, say a RAM, a non-volatile writable part, e.g., Flash. Storage may comprise a non-volatile non-writable part, e.g., ROM, e.g., storing part of the software.

In the various embodiments of communication interface 113, the communication interface may be selected from various alternatives. For example, the interface may be a network interface to a local or wide area network, e.g., the Internet, a storage interface to an internal or external data storage, an application interface (API), etc.

The device 110 may communicate internally, with other systems, with other devices, external storage, input devices, output devices, and/or one or more sensors over a computer network. The computer network may be an internet, an intranet, a LAN, a WLAN, a WAN, etc. The computer network may be the Internet. The device 110 comprise a connection interface which is arranged to communicate within device 110 or outside of device 110 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 113 may be used to send or receive digital data. For example, communication interface 113 may be used for, e.g., transmitting data, e.g., sending processed data and results from the processor to external devices or systems for further analysis, storage, and/or real-time monitoring; receiving instructions, e.g., remote control and configuration of the system parameters, such as adjusting the stimulus generator, noise generator, or processing algorithms, etc.

Brain scanning device 110 may have a user interface, which may include well-known elements such as one or more buttons, a keyboard, display, touch screen, etc. The user interface may be arranged for accommodating user interaction for, e.g., initiating a scanning, performing real-time monitoring, adjusting of scanning parameters and/or stimuli, etc.

The execution of device 110 may be implemented in a processor system 111. The device 110 may comprise functional units to implement aspects of embodiments. The functional units may be part of processor system 111. For example, functional units shown herein may be wholly or partially implemented in computer instructions that are stored in a storage of the device and executable by processor system 111.

Processor system 111 may comprise one or more processor circuits, e.g., microprocessors, CPUs, GPUs, etc. Device 110 may comprise multiple processors. A processor circuit may be implemented in a distributed fashion, e.g., as multiple sub-processor circuits. For example, device 110 may use cloud computing.

Typically, the brain scanning device 110 comprises one or more microprocessors which executes appropriate software stored at the device; for example, that software may have been downloaded and/or stored in a corresponding memory, e.g., a volatile memory such as RAM or a non-volatile memory such as Flash.

Instead of using software to implement a function, device 110 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, brain scanning device 110 may comprise circuits, e.g., for cryptographic processing, and/or arithmetic processing.

In hybrid embodiments, functional units are implemented partially in hardware, e.g., as coprocessors, e.g., arithmetic coprocessors, and partially in software stored and executed on the device.

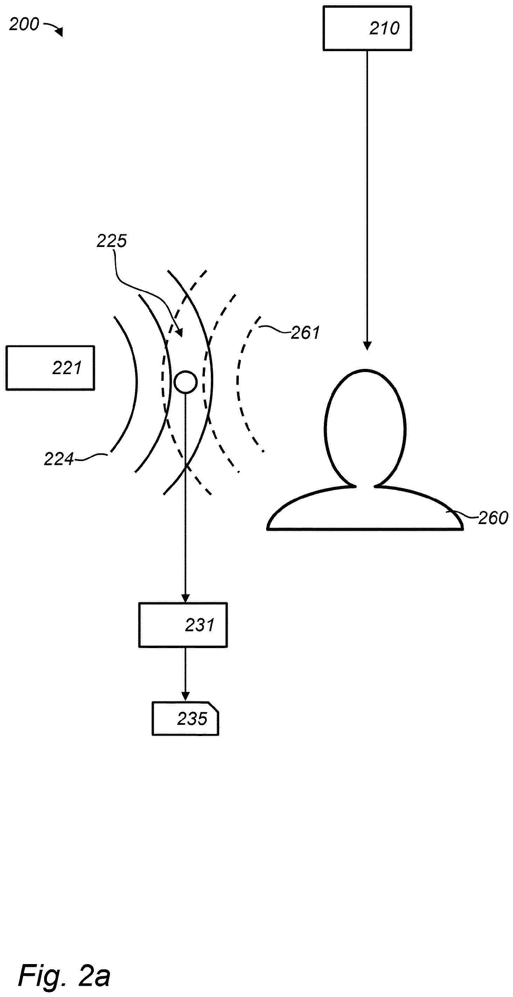

**Figure 2a** schematically shows an example of an embodiment of a brain scanning system 200.

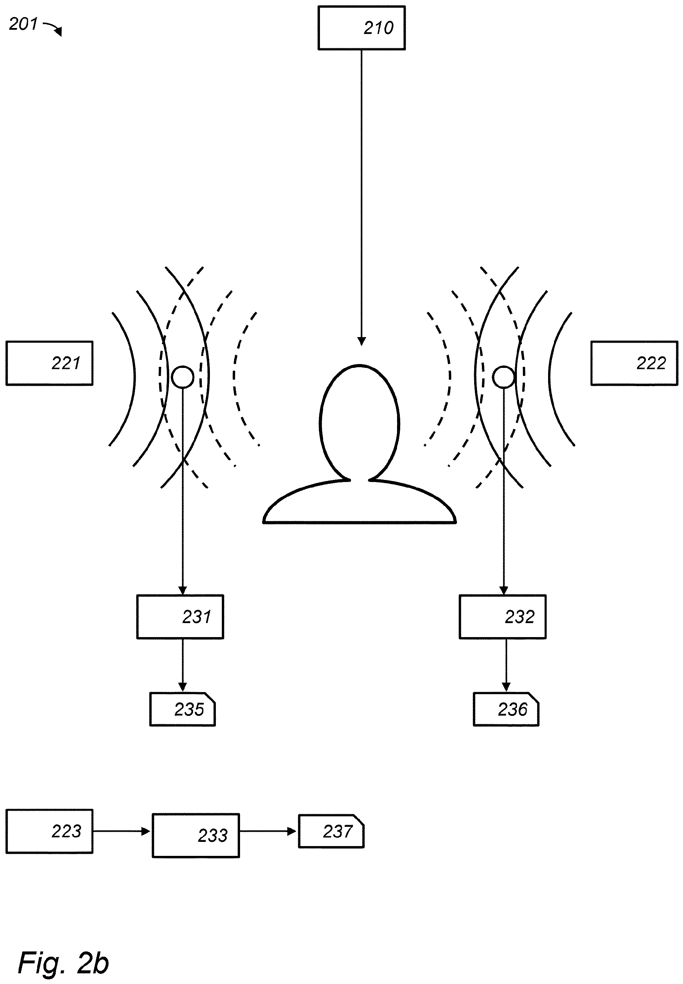

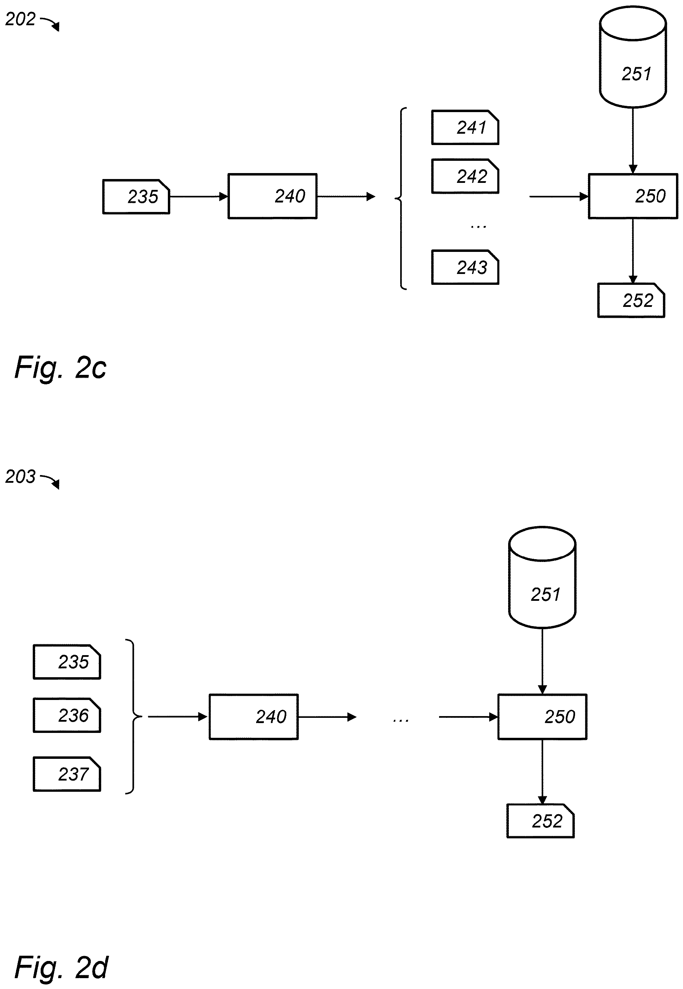

Shown in figure 2 is a human subject 260. Brain scanning system 200 is configured to detect electromagnetic responses generated by a human brain of subject 260 non-invasively.

### Stimulus Generator

Brain scanning system 200 comprises a stimulus generator 210. The stimulus generator is configured to present, at least, an auditory stimulus to the subject during or before scanning of the subject. Stimulus system 210 may be configured to deliver various stimuli to a subject's brain at specific time intervals. The brain activity and associated electromagnetic radiation thereof depend, at least in part, on the stimulus. Accordingly, the electromagnetic radiation generated by the subject's brain in after and/or during the stimulus is also referred to herein as the response.

Various types of simulation are possible, including auditory and visual stimulus. Interestingly, given a different stimulus, a different brain response is obtained which is reflected in a different interfered noise signal. Although, the differences when using different stimuli are small, a difference between the corresponding interfered noise signal can be detected, e.g., using the signal processing discussed herein.

The stimulus causes a particular brain response in the subject which in turn results in an interfered noise field. In a sense the stimulus can be regarded as the question and the response as represented in the measured interfered noise field as the answer to the question. Changing the sound and/or colors in the stimulus may then be regarded as asking a different question. Note that some time may elapse between questions and answer.

### Auditory Stimuli

The inventors found that auditory simulation is especially determinative for the response. The stimulus generator may comprise an audio module for the generation of the auditory stimulus. The auditory stimulus may be presented to the subject through one or more audio speakers.

Various auditory stimuli are possible. For example, in an embodiment, the stimulus generator is configured to generate the auditory stimulus with an ascending and/or descending band of audio frequencies. The auditory stimulus may be represented through overtones, making them audible.

For example, the stimulus generator may define a primary sound signal and obtain therefore one or more corresponding overtones, the overtones forming a secondary sound signal. In an embodiment, the secondary sound signal is included in a sound signal played to the subject. The primary sound signal may or may not be included in the played sound signal. In an embodiment, the primary sound signal consists of pure sine frequencies. The one or more overtones may have a frequency which is a multiple of the frequency of the primary sound signal. Typically, multiple overtones are included in the secondary sound signal. Including the secondary sound signal helps to make the primary sound signal more perceptible to the human ear. By better engaging the auditory pathways of the brain, a more precise brain responses during scanning is obtained.

In an embodiment, the stimulus generator is configured to generate an auditory signal according to a pure sine wave.

In an embodiment, the stimulus generator is configured to provide alternating and/or sequential stimulation of the two brain hemispheres of the subject. Typically, two speakers will be used, positioned to deliver auditory stimuli independently to each hemisphere, e.g., opposite each other at different sides of the head of the subject. By using stereo sound, or by alternating the sound between the left and right speakers, or by sequentially presenting the auditory stimulus on one hemisphere followed by the other, the system engages both sides of the brain. Such an auditory stimulus was found to be effective in eliciting a good response from the subject, presumably by triggering brain activity with a strong electromagnetic signature.

In an embodiment, a rhythmic auditory stimulation is used, prior or during the brain scanning. During this process, the subject is exposed to auditory stimuli, which can be delivered through two speakers positioned at different sides of the head. These speakers may emit the same or different signals.

For example, for rhythmic auditory stimulation sounds may be produced at regular intervals. The frequency of these sounds can vary, often ranging from low to high frequencies, or vice versa. The auditory stimuli can include repeating variations in volume and/or repeating frequency modulation, e.g., where the sound alternates in intensity and/or pitch. These rhythmic variations help trigger a measurable brain response.

For example, one speaker may emit a low-frequency sound while the other emits a high-frequency sound, or both speakers may alternate between different frequencies and volumes in a predetermined pattern.

In an embodiment, the stimulus generator uses amplitude modulation to vary the volume of the auditory stimuli, creating a rhythmic pattern that alternates between louder and softer sounds. Alternatively, or in addition, frequency modulation may be used where the pitch of the sounds changes in a repeating cycle, moving from lower to higher frequencies and back again. These modulated sounds can be presented alternately to each ear, or sequentially.

### Visual Stimuli

In an embodiment, the stimulus generator is further configured to present a visual stimulus to the subject during or before scanning of the subject. Visual stimulus is preferably used in addition to auditory stimulus, as it was found in practice that a worse response is used when using only a visual stimulus, without an auditory component. Nevertheless, in principle it is possible to create an embodiment of the brain scanning system using only visual stimulus. For example, the stimulus generator may be configured to deliver a combination of auditory and visual stimuli to a subject's brain at specific time intervals.

For example, the visual stimulus may be presented using a display. The display may be integrated in a helmet, such as a VR helmet.

Like the auditory signals, the visual stimulus may also comprise a rhythmic visual stimulus, e.g., so-called rhythmic photo stimulation.

A rhythmic audio and/or visual stimulus may comprise patterned variations in a stimulus. For example, the stimulus may vary in a consistent and repeatable pattern over time. This includes, but is not limited to, variations in amplitude (volume), frequency (pitch), timing, color, brightness, or any combination thereof, presented in a periodic or quasi-periodic manner.

It was found that additional visual stimulation helps to receive more accuracy on the brain feedback.

In an embodiment, the visual stimulation is synchronized to the audio stimulus. For example, colors may be shown with colors that are associated with frequencies in the audio signals on a display, e.g., a computer screen, watch by the subject.

In an embodiment, stimulus generator 210 is configured to retrieve a prerecorded auditory and/or visual stimulus from storage. Stimulus generator 210 is configured to present the auditory or auditory plus visual stimulus to the subject. In an embodiment, stimulus generator 210 is configured to generate auditory and/or visual stimuli from mathematical algorithms. These generated stimuli can include complex waveforms, such as those derived from Fourier transformations or other mathematical models, providing precise control over the frequency, amplitude, and duration of the auditory signals presented to the subject. For example, an auditory stimulus may comprise one or more pure sinewave. For example, an auditory stimulus may comprise multiple overtones generated for a pure sinewave. The stimulus generator comprises a digital signal processor (DSP) configured for the above.

The stimulus generator may be synchronized with one or more noise generators, further discussed below. For example, the stimulus generator may be configured to operate during or before the scanning of the subject. To deliver various stimuli at specific time intervals, the stimulus generator may comprise a timing control module. This module schedules the delivery of auditory and/or visual stimuli at predetermined intervals.

### Noise Generator

Brain scanning system 200 comprises one or more noise generators. Figure 2a shows one noise generators 221. A noise generator is configured to generate an electromagnetic noise field, while positioned in proximity to the subject's head. During electromagnetic noise field generated by noise generator 221 interacts with the brain's electromagnetic field, resulting in an interfered noise signal.

Shown in figure 2a is noise generator 221 positioned next to the head of subject 260. Noise generator 221 generates and radiates an electromagnetic noise field: noise generator electromagnetic noise field 224. At the same time, the subject's brain also produces an electromagnetic field, in particular as a result of stimulation of the subject, e.g., rhythmic simulation: subject electromagnetic field 261. These two electromagnetic field interfere resulting in an interfered electromagnetic noise field 225. Interfered electromagnetic noise field 225 is measured-symbolically represented in figure 2a with a small circle-and provided to an analog-to-digital converter 231 to obtain digital sequence 235 that represents the interfered electromagnetic noise field 225.

The interfered electromagnetic noise field 225 is measured close near the subject's head.

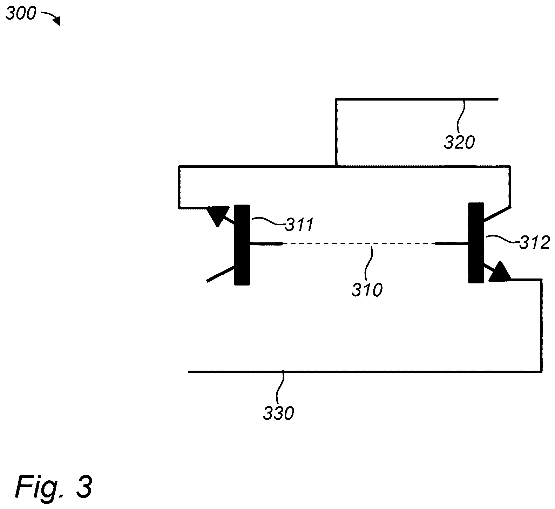

Measuring the interfered electromagnetic noise field 225 may use a separate electromagnetic sensor, which may be independent of the noise generator, however in an advantageous embodiment generating and measuring the electromagnetic field is integrated in one device, and example thereof is shown with reference to figure 3. The noise field of the noise generator is interfered with by the brain and sent to an analog-to-digital converter (ADC).

Whether an independent sensor is used or whether the noise generator itself is configured for sensing (as is preferred), sensor or noise generator would be positioned in proximity to the scalp of the subject. Preferably, at most 2 cm distance, more preferably at most 1 cm distance, more preferably, at most 1 mm distance.

Figure 2a shows one noise generator, though multiple noise generators may be used, typically arranged symmetrically near the subject's head.

In an embodiment, the noise generator produces white noise, e.g., having a constant power spectral density in a particular range. In an embodiment, the electromagnetic noise field of the noise generator has a frequency range of at most 12 Hz, and/or at least 0.5 Hz, in particular, wherein the range is from 1.8 Hz to 8.2 Hz.

**Figure 2b** schematically shows an example of an embodiment of a brain scanning system 201. Brain scanning system 201 is similar to brain scanning system 200 but includes a reference noise generator 223. Brain scanning system 201 preferably uses at least two noise generators placed in direct proximity to the subject's scalp.

Shown in figure 2b are two noise generators: noise generator 221 and noise generator 222. Each generates an electromagnetic noise field which interacts with the brain's electromagnetic field, resulting in an interfered noise field and corresponding interfered noise signal associated with that noise generator. Each interfered noise signal is measured, preferably by the noise generator itself, and led to an analog-to-digital converter. As shown each noise generator had a dedicated analog-to-digital converter: analog-to-digital converter 231 to convert the interfered noise signal to a digital sequence 235, and analog-to-digital converter 232 to convert the interfered noise signal to a digital sequence 236.

Brain scanning system 201 comprises a reference noise generator 223. The reference noise generator is also configured to generate an electromagnetic noise field, but the reference noise generator is not positioned in proximity to the subject's head. Accordingly, the reference noise generator does not result in an interfered noise signal. An analog-to-digital converter 233 converts the interfered noise signal to a digital sequence 237.

The noise generators including the reference noise generator are of the same design and operation so that, assuming the absence of interaction with the brain's electromagnetic field, they would result in noise with the same, or similar, statistical properties. For example, taking any two noise generators including the reference noise generator, a Kolmogorov-Smirnov test for a significance level of α=0.05, more preferably α=0.01, would show that the two noise generators have the same distribution.

Brain scanning system 200 and 201 could be implemented on a system such as system 100. They could be implemented on a computer system comprising one more microprocessors, such as the MEGA 2560 microprocessor. In an embodiment, brain scanning system may comprise one or more of: a noise filter, a power supply decoupling, an electromagnetic shielding to block external electromagnetic interference. High-Frequency Noise Filter reduces interference from unwanted high-frequency signals. The filter may be applied to the analog interfered noise signal. The filter also improves conversion to a digital sequence. Filtering may also be performed after ADC conversion. Power Supply Decoupling reduces power supply noise and voltage fluctuations, this reduces unwanted artefacts in the inferred noise signal. Electromagnetic Shielding blocks external electromagnetic interference, protecting the system from environmental noise. Note that also the reference noise generator may be protected by one or more of the measures.

Brain scanning system 201 as shown comprises a dedicated analog-to-digital converter for each noise generator, although this is preferred, it is not strictly necessary. Fewer, possibly even a single ADC, could be employed by multiplexing the signals.

In an embodiment, the brain scanning system comprises three noise generators: two for placing in proximity to the subject scalp, and one as a reference noise generator. More than two noise generators for proximity are possible. Two of the at least three noise generators may be positioned at the left and right brain hemispheres. The analog-to-digital converter(s) are configured to convert the interfered noise signals from each hemisphere separately.

There are several ways to package the noise generator(s) that are arranged for placement near the subject's skull.

In an embodiment, at least one of the noise generator is comprised in, e.g., attached to, a headband. In this embodiment separate speaker(s) and optionally display(s) are used for stimulation.

In an embodiment, at least one of the noise generator is comprised in a headphone. The headphone is arranged to keep the noise generator in close contact with the head. The headphone speakers may be used for auditory stimulation, making this an advantageous combination. For example, a noise generator may be integrated at each side of the headphone. In an embodiment, one or more noise generator are placed next to each temporal bone on opposite sides of the head. Note that, although placement of the noise generator in proximity with the scalp is preferred, direct contact is not needed.

In an embodiment, at least one of the noise generators is comprised in a VR headset. The headset is arranged to keep the noise generator in close contact with the head. The headset comprises speaker(s) which may be used for auditory stimulation, and a display which may be used for visual stimulation.

Figure 2c schematically shows an example of an embodiment of a brain signal processing system 202. Figure 2c shows a processing system for use with a brain scanning system with at least one noise generator, such as brain scanning system 200. The processing system of figure 2c is typically integrated with the brain scanning system, although this is not necessary.

Figure 2c shows a digital sequence 235, such as obtained from an ADC 221. Digital sequence 235 represents the interfered noise signal measured near the subject's scalp. Digital sequence 235 is processed by a frequency processing unit 240 to derive therefrom a series of bands. Each band corresponds to a specific ranges of frequencies within the overall signal. Shown in figure 2c are bands 241-243.

For example, in an embodiment, the electromagnetic noise field generated by the one or more noise generators have a frequency range of at most 12 Hz, and/or at least 0.5 Hz; in particular, wherein the range is from 1.8 Hz to 8.2 Hz. The frequency processing of the digital sequence may mirror this. For example, the frequency processing may be configured to detect frequency peaks in the series of bands within a frequency range of at most 12 Hz, and/or at least 0.5 Hz, in particular, wherein the range is from 1.8 Hz to 8.2 Hz.

For example, in an embodiment, the information from up various brain rhythms is processed, e.g., the 11 brain rhythms known as (γ, β, α, κ, µ, τ, λ, σ, τ, θ, δ). Each of these rhythms is in essence sending information, which is causing a specific interference in the electromagnetic noise field from the generator.

The frequence values obtained from the frequency processing, e.g., the activity in each of the bands, is now correlated with reference samples from a database.

Brain signal processing system 202 comprises a correlation unit 250. Correlation unit 250 is configured to take as input the output of frequency processing unit 240, e.g., bands 241-243, and on the compare it to each of a series of reference samples. For example, reference database 251 may comprise multiple reference samples. Correlation unit 250 provides as output a correlation result 252. Correlation result 252 may comprise, e.g., the reference sample that most resembles the current sample, or a top-k, wherein say k at most 5, or at most 10, of samples that most resemble the current sample. Resemblance may be measured by computing correlation, wherein a low correlation is associated with high resemblance; for example, a threshold may be chosen wherein a correlation between the threshold indicates a match. The precise value of the threshold depends on various factors, for example, the number of bands, the number of intervals, and the resolution of the ADCs used. However, a threshold can be established empirically. This may comprise comparing samples obtained from the same individual and the same stimulus, say, obtained on different days-wherein the threshold is chosen sufficiently high so that the system will indicate matches. Likewise, samples obtained from different individuals may be compared, where it is known that the individuals likely do not match, due to a different medical assessment.

For example, two sets may be formed. A set *A* with correlation values that should match, and a set *B* with correlation values that should not match. A threshold may be computed as the midpoint between the averages of the two sets, e.g., *t* = 0.5 * *µ_{A} +* 0.5 * *µ_{B}*. As better information may be available for matching than non-matching correlation values, the average may be weighted, e.g., *t = αµ_{A}* + (1 - *α*)*µ_{B}*, with 1 > *α* > 0.5, for example, *α* = 2/3. Alternatively, an ROC curve analysis may be performed to further optimized the threshold. Further correlation schemes may be used, e.g., employing artificial intelligence to compute a correlation score.

A possible output may be that none of the reference samples match the current sample; as the size of the reference database grows this is increasingly unlikely. In an implementation of the system build by the inventors, over 10000 reference samples are recorded, and a match is virtually certain.

These reference samples, stored in reference database 251, were previously obtained from other individuals who were preferably triggered with equivalent stimuli as the current subject, e.g., the same stimulus. Along with the reference samples, details about the reference subjects are also recorded. A match between a reference sample and the current subject sample indicates a likelihood that the recorded details also apply to the current subject. For example, such a recorded detail may be sleeplessness or anxiety.

The procedure may be repeated for different stimuli. This is important as the inventors found that different stimuli tend to reveal information about different things. For example, a stimulus configured to obtain information on sleeplessness may be different from a stimulus configured to obtain information on migraines, etc.

In an embodiment, the stimulus generator is configured to present an auditory stimulus to the subject either during or before the scanning process. This stimulus can be in the form of a noise or an image. A stimulus may be optimized to obtain information about a range of topics; for example, stimulus may be optimized to obtain information related to a particular organ, such as the liver. The presentation of the stimulus triggers a response in the brain. In addition to audio, the stimulus may also comprise a visual component.

One or more noise generators that each create an electromagnetic noise field are positioned in proximity to the subject's head. During operation, the generated electromagnetic noise field interacts with the brain's electromagnetic field, resulting in an interfered noise signal. Frequency processing of a digital sequence representing the interfered noise signal results in a series of frequency bands, e.g., in the range of 1.8 to 8.2 Hz.

The processor then correlates the derived activity with a database of reference samples to identify specific patterns. Different patterns with varying frequency peaks emerge based on the brain's response to the stimulus. The system is designed to detect these patterns. The inventors found that these patterns typically show repetitions, especially at the beginning and end of the sequence. Finding such repetitions is understood to be an indicator for accurate results.

To improve accuracy the system may be calibrated. Calibration may improve the system's results due to potential machine inaccuracies and the inherent variability in Earth's electromagnetic field. This calibration process may comprise adjusting system parameters (in particular parameters related to the noise field amplitude) until a detected pattern is repeated a certain number of times, e.g., at least three times. In an embodiment, calibration is performed for each noise generator. For example, calibration may be performed both on the left and the right sides of the brain.

**Figure 2d** schematically shows an example of an embodiment of a brain signal processing system 203. System 203 is arranged for processing multiple digital sequences coming from the same subject 260. For example, system 203 may be used with scanning system 201 and its variants, e.g., as described with reference to figure 2b.

Multiple digital sequences are received from multiple noise generators. Shown in figure 2d are digital sequences 235 and 236 received from ADCs connected to noise generators 231 and 232, respectively. Furthermore, a reference digital sequence 237 is received. Reference digital sequence 237 is optional, but was found beneficial in processing the digital sequences. Reference digital sequence 237 is received from an ADC connected to reference noise generator 223. It is possible to combine various numbers of noise generators with a reference noise generator, e.g., one noise generators and one reference noise generator, or multiple noise generators and one reference noise generator. In an embodiment, two noise generators and one reference noise generator were used.

In an embodiment, a processor of system 203 synchronizes the received digital sequences, e.g., sequences 235, 236, 237. In particular, a separate left and right brain digital sequence and reference digital sequence are synchronized.

For example, each digital sequence may be time-stamped at the moment of conversion to ensure accurate temporal alignment. An auxiliary clock module can provide precise time stamps. Further synchronization may be performed after conversion to a digital sequence. For example, a processor may perform a cross-correlation analysis on the time-stamped digital sequences. The analysis may comprise measuring a similarity between two sequences as a function of the time-lag applied to one of them. Evaluating similarity may comprise computing a correlation. The time-lag with maximal similarity may be applied as a time shift to one sequence to align the sequences.

After the optional synchronization, all digital sequences, e.g., sequences 235, 236, and 237, are frequency processed, e.g., by frequency processing unit 240, resulting in multiple series of bands, e.g., as described with reference to figure 2c.

In an embodiment, the reference bands obtained from the reference noise generator are used to identity patterns in the other bands that are due to the brain's response to the stimulus. For example, in an embodiment, the reference bands are subtracted from the bands obtained from the other noise generators.

The cleaned up bands may be correlated with the reference samples. For example, a noise generator on the left temporal lobe may be correlated with reference samples that were also obtained with from the left temporal lobe but from other subjects with a known medical background.

For example, in an embodiment with two noise generators positioned at opposite hemispheres, and one reference noise generator; each noise generator may be compared individually with reference samples specific for the location of the noise generator on the subject's head.

In an embodiment, having *n* > 2 noise generators, giving bands *a*₁*, ... , aₙ,* wherein each *aᵢ* stands for a series of bands, a correlation may be done for any subset of the bands, e.g., a correlation may be done for (*a*_{*i*₁} , *a*_{*i*₂} , ... , *a_{iₖ}* )*,* wherein the subscripts identify some subset of the bands. In particular, for *n* = 2 noise generators, a correlation may be done for (*a*₁, *a₂*)*.* In these cases, the database may comprise multiple series of bands for the subjects in the database.

In an embodiment, a mathematical function is applied to the bands before correlation. In particular, given two noise generators placed at opposite ends of a subject head, in particular the temporal lobes, a correlation may be done for *a*₁ - *a*₂.

As said, the system, e.g., the scanning and processing system, may be calibrated. The calibration may comprise comparing the digital sequences for both the left and right hemispheres. The calibration increases coherency between the left and right brain signals. For example, the configuration may be terminated when the two sequences from both sides have a coherency above a predetermined threshold, or if said coherency can no longer be improved.

In experiments, the inventors found that a scanning system as described with reference to figure 2d, using two noise generators and one reference noise generators could predict medical information on a variety of topics, including psychological and/or physiological states or characteristics of the subject. Examples include: cognitive load, emotional state, stress levels, sleep patterns, neurological disorders, attention levels, and meditative states. This list is not exhaustive, various other state types may be also, or instead, be detected and/or quantified using an embodiment as described herein.

In certain embodiments, the operation of the claimed invention may be understood in the context of interoceptive processes, where an embodiment of the brain scanning system could align with the body's internal sensory feedback mechanisms. While interoception offers a useful framework for interpreting the invention's operation, it should be noted that the claimed invention is not limited to mechanisms solely dependent on interoceptive processes.

Interoception is the scientific field of research that examines the exchange of information between the brain and the body, sometimes referred to as the mind-body relationship. Various studies have proven that there is a loop between the brain and the body: signals from the body end up in the brain, and signals from the brain end up in the body. It implies that a person's experiences, which cause signals in the brain, will have an impact on the body.

Interoception refers to the process by which the nervous system senses, interprets, and integrates signals originating from within the body, particularly from the internal organs, such as the heart, lungs, stomach, and intestines. Although interoception is a relatively new field, several studies have proven that experiences, even if initially processed in the brain, affect the body. Interoception includes the perception of physiological states, including hunger, thirst, heart rate, respiration, and internal pain, as well as more subtle sensations like gut feelings or the sense of physical well-being or discomfort.

Several brain areas process interoceptive signals and contribute to the conscious awareness of the body's internal states, influencing decision-making, emotional regulation, and overall well-being.

Interoception has been linked to various mental health conditions, including anxiety, depression, and eating disorders, where interoceptive processing might be altered or dysfunctional.

The exchange process between brain and body has been investigated, among others, by N. P. Bekhtereva. We refer to the English language overview article "Dangers and Opportunities for Change from a Physiologist's Point of View", and the references cited therein. By studying electrical potentials in discrete zones of the human brain, it was shown that sustained negative emotions, such as fear of approaching disaster, can unbalance the brain's normal state, causing electrical levels to rise too high or fall too low. Quantitative measurement of parameters of brain functioning was obtained using forty to seventy gold electrodes implanted in the human brain. Using a brain scanning system according to an embodiment, it is possible to obtain information on the brain using non-invasive methods.

In an embodiment, the brain scanning system comprises: a stimulus system configured to deliver a combination of field, auditory, and optionally visual stimuli to a subject's brain at specific time intervals. Furthermore, the brain scanning system comprises
- at least three noise generators used to generate an electromagnetic noise field, with at least two of the noise generators arranged symmetrically near the subject's head, wherein during operation of the brain scanning system, the electromagnetic noise field generated thereby combined with visual signals and sound frequency signals interacts with the electromagnetic field of the brain, resulting in the generation of brain response signals in analog form.
- an analog-to-digital converter for converting the received brain response signals (interference noise) expressed in analog form into a digital sequence.
- a processor configured to receive and process a digital sequence of signals received from the ADC to convert them into a number of ranges for the purpose of further correlation of the received information with a database of reference samples.

**Figure 3** schematically shows an example of an embodiment of a noise generator 300.

Noise generator 300 comprises an interference injection point 310. Interference injection point 310 receives the electromagnetic field received from the subject's head, e.g., generated by his/her brain. Noise generator 300 also generates an electromagnetic noise field itself, in the example, of figure 3, by two transistors: transistors 311 and 312, which is combined with the electromagnetic field received at interference injection point 310.

The combined electromagnetic field is made available on an amplification connection 320, where an amplification circuit (not separately shown in figure 3) may be connected for amplification of the combined electromagnetic field. The output of the amplification circuit is then passed on to an ADC to obtain a digital sequence. Amplification is optional and may be omitted, e.g., if the ADC does not require it; in that case amplification connection 320 may be used as an output, e.g., to the ADC or to a further circuit for further signal processing.

Also shown in figure 3 is a power connection 330 for powering the noise generator.

Transistors 311 and 312 are configured to generate the noise field. For example, these may be an NPN bipolar junction transistor (BJT). For example, they may be transistors of type 2N3904.

In an embodiment, interference injection point 310 is a wire arranged for positioning proximate to the subject's head. The wire may be arranged in various ways, as desired, e.g., as a line, a coil, and the like. Note that the interference injection point 310is part of the mechanism for generating the electromagnetic noise field, but is also arranged for reception of the brain's electromagnetic field response. In an embodiment, the electromagnetic noise field has a frequency range of at most 12 Hz, and/or at least 0.5 Hz, in particular, wherein the range is from 1.8 Hz to 8.2 Hz.

Best results for the length of the wire is 5 mm. In an embodiment, the wire is between 4.5 and 5.5 mm.

In an embodiment, noise generator 300 generates electronic noise through a combination of semiconductor components, in this example two transistors and arranged to receive an external electromagnetic interference at interference injection point 310.

Noise generator 300 comprises a noise generator stage. In this embodiment, the noise generator stage comprises two NPN transistors: transistors 311 and 312, configured with their bases connected at the interference injection point (IIP) 310. In this configuration, the transistors operate in a reverse-biased mode, so that the so-called avalanche breakdown phenomenon, generates electronic noise. The interference injection point comprises a connection between the bases of the two transistors. The design intentionally exposes this point to external electromagnetic fields, which introduces the brain's electromagnetic field into the noise signal generated by the transistors, resulting in an interfered noise signal. Noise generator 300 is arranged for the interference injection point to be close to the subject's head.

Noise generator is configured to connect to an amplification stage. The amplifier could be integrated in the noise generator, or could be external to the noise generator. For example, the amplifier could comprise one or more further transistors, e.g., an NPN transistor configured as an amplifier. The amplifier receives the noise signal from the noise generation stage and amplifies it for subsequent processing and measurement.

For example, further processing may comprise a voltage divider for scaling the signal to a voltage level suitable for input to an analog-to-digital converter (ADC).

Noise generator 300 may be powered at 330, preferably using a regulated supply.

In an embodiment noise generator 300 comprises filtering and stabilization circuits. For example, noise generator 300 may comprise a high-frequency filtering circuit for filtering the generated noise field and/or interfered noise field. The high-frequency filtering circuit may comprise a non-polarized capacitor. For example, noise generator 300 may comprise a filtering circuit for filtering the power supply, e.g., to smooth out power supply fluctuations. The latter filtering circuit may comprise a polarized capacitor. The circuit may comprise multiple ground points to ensure a common ground reference.

The processed interfered noise signal may be sent to an analog input of an ADC, e.g., comprised in a microcontroller to convert the analog noise signal into a digital sequence.

Noise generator 300 may comprise a tuning circuit for tuning the signal strength of the generated electromagnetic noise field to that of the received electromagnetic noise field. For example, this may be done during a calibration method.

The numbered clauses below represented beneficial embodiments of the noise generator.

Clause 1. A noise generator configured for non-invasive detection of electromagnetic responses generated by a human brain of a subject, the noise generator being configured to generate an electromagnetic noise field, the noise generator being arranged for positioning in proximity to the subject's head, wherein during operation of the noise generator the generated electromagnetic noise field interacts with the brain's electromagnetic field, resulting in an interfered noise signal.

Clause 2. The noise generator of any one of the preceding clauses, further comprising an interference injection point configured to receive the electromagnetic field generated by the subject's brain and combine it with the generated electromagnetic noise field.

Clause 3. The noise generator of any one of the preceding clauses, wherein the interference injection point is a wire arranged for positioning proximate to the subject's head

Clause 4. The noise generator of any one of the preceding clauses, wherein the electromagnetic noise field has a frequency range of at most 12 Hz, and/or at least 0.5 Hz, in particular, wherein the range is from 1.8 Hz to 8.2 Hz.

Clause 5. The noise generator of any one of the preceding clauses, further comprising a tuning circuit for tuning the signal strength of the generated electromagnetic noise field to that of the received electromagnetic noise field during calibration of the noise generator.

Clause 6. The noise generator of any one of the preceding clauses, wherein the noise generator stage comprises two transistors configured with their bases connected at the interference injection point, and the transistors operate in a reverse-biased mode to generate electronic noise via the avalanche breakdown phenomenon.

Clause 7. The noise generator as in Clause 6, wherein the transistors are NPN bipolar junction transistors (BJTs).

Clause 8. The noise generator of any one of the preceding clauses, wherein the transistors are arranged to receive an external electromagnetic interference at the interference injection point, thereby introducing the brain's electromagnetic field into the noise signal generated by the transistors.

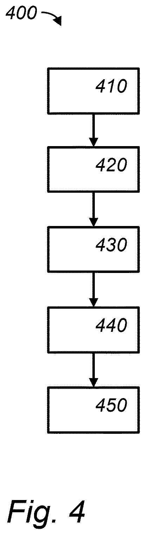

Figure 4 schematically shows an example of an embodiment of a brain scanning method 400. Method 400 may be computer implemented. Then brain scanning method of figure 4 is for non-invasive detection of electromagnetic responses generated by a human brain of a subject. Brain scanning method 400 comprises:
- presenting (410) an auditory stimulus to the subject during or before scanning of the subject,
- generating (420) an electromagnetic noise field in proximity to the subject's head, wherein during operation of the brain scanning system the generated electromagnetic noise field interacts with the brain's electromagnetic field, resulting in an interfered noise signal,
- receiving (430) and converting the interfered noise signal into a digital sequence,
- performing (440) frequency processing on the digital sequence, and deriving therefrom activity in a series of bands,
- correlating (450) the derived activity with a database of reference samples.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform an embodiment of method 400. Software may only include those steps taken by a particular sub-entity of the system. The software and/or other data according to an embodiment may be stored in a non-transitory storage medium, such as a hard disk, a floppy, a memory, an optical disc, read only memory, random access memory, CD-ROMs, magnetic tape, optical data storage devices, etc. Transitory signals and carrier waves are excluded from non-transitory media.

The software may be sent as a transitory signal along a wire, or wireless, e.g., sent as a transitory signal over a data network, e.g., the Internet. For example, signals and/or carrier waves may serve as a transitory medium for carrying information. For example, a modulated electromagnetic wave may carry a signal bearing the software and/or other data according to an embodiment.

The software may be made available for download and/or for remote usage on a server. Embodiments of the method may be executed using a bitstream arranged to configure programmable logic, e.g., a field-programmable gate array (FPGA), to perform an embodiment of the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units, and/or parts of at least one of the systems and/or products set forth.

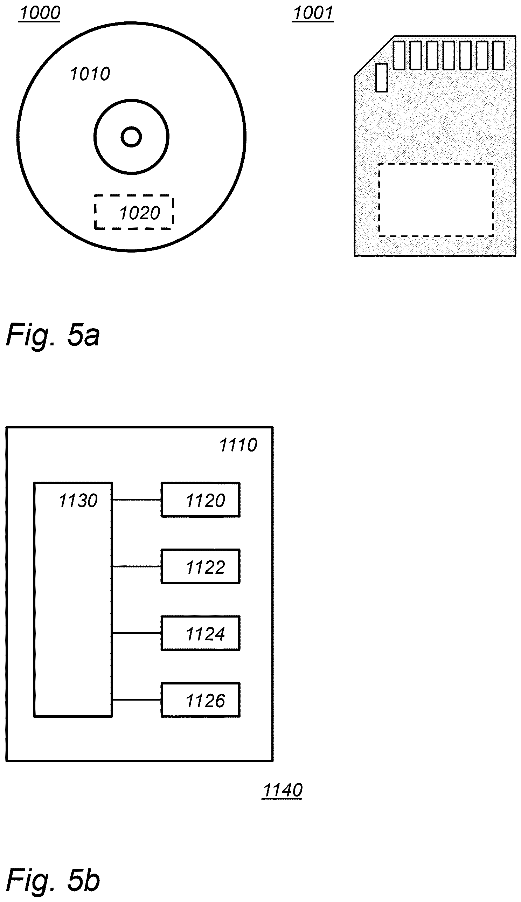

**Figure 5a** shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a brain scanning method, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform an embodiment of said brain scanning method.

**Figure 5b** shows in a schematic representation of a processor system 1140 according to an embodiment for brain scanning. The processor system comprises one or more integrated circuits 1110. The architecture of the one or more integrated circuits 1110 is schematically shown in Figure 5b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1140 may be arranged for contact and/or contact-less communication, using an antenna and/or connectors, respectively.

For example, in an embodiment, processor system 1140, e.g., the brain scanning device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While system 1140 is shown as including one of each described component, the various components may be duplicated in various embodiments. For example, the processing unit 1120 may include multiple microprocessors that are configured to independently execute the methods described herein or are configured to perform elements or subroutines of the methods described herein such that the multiple processors cooperate to achieve the functionality described herein. Further, where the system 1140 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A brain scanning system configured for non-invasive detection of electromagnetic responses generated by a human brain of a subject, the brain scanning system comprising:
- a stimulus generator configured to present an auditory stimulus to the subject during or before scanning of the subject,
- one or more noise generators configured to generate an electromagnetic noise field, the noise generator being arranged for positioning in proximity to the subject's head, wherein during operation of the brain scanning system the generated electromagnetic noise field interacts with the brain's electromagnetic field, resulting in an interfered noise signal,
- an analog-to-digital converter to convert the interfered noise signal received into a digital sequence,
- a processor configured to
- obtain the digital sequence,
- perform frequency processing on the digital sequence, deriving therefrom activity in a series of bands,
- correlate the derived activity with a database of reference samples.

2. The brain scanning system according to claim 1, wherein the stimulus generator is further configured to present a visual stimulus to the subject during or before scanning of the subject.

3. The brain scanning system according to any one of the preceding claims, wherein the system comprises at least three noise generators.

4. The brain scanning system according to claim 3, wherein two of the at least three noise generators are positioned at the left and right brain hemispheres, the analog-to-digital converter being configured to convert the interfered noise signals from each hemisphere separately, the processor being configured to synchronize the corresponding digital sequences.

5. The brain scanning system according to claim 4, wherein the system is configured for calibration of the digital sequence on both the left and right hemispheres, the calibration increasing coherency between left and right brain signals.

6. The brain scanning system according to any one of the preceding claims, wherein
- at least one of the noise generators are integrated into a headphone comprising a speaker for providing the auditory stimulus, and/or
- at least one of the noise generators are integrated into a VR headset, the VR headset comprising a display for providing a visual stimulus to the subject during or before scanning of the subject.

7. The brain scanning system according to any one of the preceding claims, wherein the stimulus generator is configured to provide alternating and/or sequential stimulation of the two brain hemispheres of the subject.

8. The brain scanning system according to any one of the preceding claims, wherein the stimulus generator is configured to generate the auditory stimulus with an ascending and/or descending band of audio frequencies.

9. The brain scanning system according to any one of the preceding claims, wherein the stimulus generator is configured to generate a rhythmic auditory and/or visual stimulus and present the stimulus to the subject during or before scanning of the subject.

10. The brain scanning system according to any one of the preceding claims, wherein
- the electromagnetic noise field has a frequency range of at most 12 Hz, and/or at least 0.5 Hz, in particular, wherein the range is from 1.8 Hz to 8.2 Hz, and/or
- the frequency processing is configured to detect frequency peaks in the series of bands within a frequency range of at most 12 Hz, and/or at least 0.5 Hz, in particular, wherein the range is from 1.8 Hz to 8.2 Hz.

11. The brain scanning system according to any one of the preceding claims, comprising one or more of: a high-frequency noise filter, a power supply decoupling, an electromagnetic shielding to block external electromagnetic interference.

12. The brain scanning system according to any one of the preceding claims, wherein the processor is further configured to identify from the correlating one or more psychological and/or physiological states or characteristics of the subject, wherein the psychological and/or physiological state comprises one or more of the following: cognitive load, emotional state, stress levels, sleep patterns, neurological disorders, attention levels, and meditative states.

13. A noise generator configured for non-invasive detection of electromagnetic responses generated by a human brain of a subject, the noise generator being configured to generate an electromagnetic noise field, the noise generator being arranged for positioning in proximity to the subject's head, wherein during operation of the noise generator the generated electromagnetic noise field interacts with the brain's electromagnetic field, resulting in an interfered noise signal.

14. A brain scanning method (400) configured for non-invasive detection of electromagnetic responses generated by a human brain of a subject, the brain scanning method comprising:
- presenting (410) an auditory stimulus to the subject during or before scanning of the subject,
- generating (420) an electromagnetic noise field in proximity to the subject's head, wherein during operation of the brain scanning system the generated electromagnetic noise field interacts with the brain's electromagnetic field, resulting in an interfered noise signal,
- receiving (430) and converting the interfered noise signal into a digital sequence,
- performing (440) frequency processing on the digital sequence, and deriving therefrom activity in a series of bands,
- correlating (450) the derived activity with a database of reference samples.

15. A transitory or non-transitory computer storage medium encoded with instructions that, when executed by one or more computers, cause the one or more computers to perform the method according to claim 14.
